# FASCICULE DE BREVET EUROPEEN

(11) **EP 2 486 019 B1**
(45) Date de publication et mention de la délivrance du brevet: **25.03.2015**
(21) Numéro de dépôt: 10761023.0
(22) Date de dépôt: 06.10.2010
(51) Int. Cl.: C07D 233/84

(54) **PROCEDE DE SYNTHESE DE LA 2-THIOHISTIDINE ET ANALOGUES**
VERFAHREN ZUR SYNTHESE VON 2-THIOHISTIDIN UND DERGLEICHEN
METHOD FOR THE SYNTHESIS OF 2-THIOHISTIDINE AND THE LIKE

(30) Priorité: 06.10.2009 FR 0956968
(43) Date de publication de la demande: 15.08.2012
(73) Titulaire: Tetrahedron, 94300 Vincennes (FR)
(72) Inventeur: ERDELMEIER, Irène, 75013 Paris (FR); DAUNAY, Sylvain, 94130 Nogent sur Marne (FR)
(74) Mandataire: Regimbeau
(86) Numéro de dépôt international: PCT/EP2010/064947
(87) Numéro de publication internationale: WO 2011/042478

(56) Documents cités:
- US-A1- 2009 093 642
- SHOSUKE ITO: "Synthesis of 2-S-cysteinylhistidine and 2-mercaptohistidine via bromolactone derivative of histidine", JOURNAL OF ORGANIC CHEMISTRY, vol. 50, no. 19, septembre 1985 (1985-09), pages 3636-3638, XP009129621, DOI: 10.1021/jo00219a044

## Description

La présente demande de brevet concerne un nouveau procédé de synthèse de la 2-thiohistidine et de dérivés apparentés.

La L-2-thiohistidine, encore appelée L-2-mercaptohistidine ou acide α-amino-2,3-dihydro-2-thioxo-1H-imidazole-4-propanoïque, répond à la formule I-1 suivante :

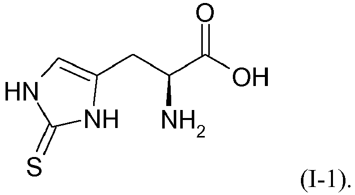

Cet acide aminé a été obtenu par hydrolyse acide de protéines à cuivre telles que l'hémocyanine de mollusque ou la tyrosinase de champignon. La L-2-thiohistidine est un bon chélateur d'ions métalliques divalents tels que Zn²⁺ ou Cu²⁺. Elle est utilisée notamment en cosmétologie, comme agent dépigmentant ou encore comme déodorant, et également comme agent antioxydant pour une utilisation pharmaceutique, cosmétique ou encore alimentaire. Au vu de l'intérêt de ce composé, plusieurs synthèses de la 2-thiohistidine ou de ses dérivés ont été décrites dans la littérature.

Ainsi, une première synthèse décrit la transformation de l'ester méthylique de la L-histidine en dérivé di-ter-butoxycarboxylé (di-Boc). Ce dernier est ensuite traité avec du chlorothionoformate de phényle qui, après traitement, conduit à un mélange d'esters méthyliques de mono- et de di-Boc-2-thiohistidine. Après déprotection, la L-2-thiohistidine est obtenue avec un rendement global de 70% (J. Xu, J. C. Yadan, J. Org. Chem. 60, 6296 - 6301 (1995*)*).

Cependant, une telle synthèse nécessite la protection du groupement amino de l'histidine qui ne peut être présent sous forme libre. De plus, le chlorothionoformate de phényle doit être préparé à partir du thiophosgène (CSCl₂), réactif toxique et difficilement disponible en grande quantité pour une utilisation à l'échelle industrielle.

Les demandes internationales WO 95/18 108 et WO 95/00 494 décrivent la synthèse de la Nα,Nα-diméthyl-2-thiohistidine ou de son ester à partir de l'ester méthylique de la Nα,Nα-diméthyl-histidine par réaction avec du chlorothioformiate de phényle, puis éventuellement hydrolyse.

La demande de brevet US 2009/093 642 décrit également une synthèse de la 2-thiohistidine (selon une méthode décrite par Heath, H. et al. J. Chem. Soc., 1951, 2215) à partir de l'histidine par ouverture du cycle imidazole puis réaction avec un thiocyanate tel que KSCN. Outre l'utilisation de grands volumes d'acide chlorhydrique, le KSCN, utilisé en milieu acide, est un réactif hautement toxique.

Shosuke Ito a également décrit une synthèse de la 2-thiohistidine à partir de l'histidine en 1985 (J. Org. Chem. 1985, 50, 3636-3638). Ainsi, l'histidine est mise en réaction avec du dibrome pour donner, vraisemblablement par l'intermédiaire d'une bromolactone, un thioéther de la 2-thiohistidine après réaction avec de la cystéine. Une hydrolyse réductrice en présence d'acide iodhydrique HI et de phosphore rouge P donne alors la 2-thiohistidine attendue et de la (D,L)-alanine. La 2-thiohistidine peut également être obtenue directement à partir de l'intermédiaire bromolactonique par réaction avec Na₂S.

Cependant, le procédé d'Ito pose, au niveau industriel, des problèmes de purification puisque deux chromatographies sur colonne sont nécessaires pour obtenir le thioéther intermédiaire, ainsi que pour obtenir le produit final, la 2-thiohistidine finale n'étant obtenu qu'avec un rendement global de 12%. En outre l'utilisation de phosphore rouge n'est pas recommandée pour une utilisation au niveau industriel, en raison de sa grande inflammabilité. De plus, de l'hydrogène est formé au cours de cette réaction entre l'acide iodhydrique HI et le phosphore rouge P (J. Organomet. Chem. 529, 295-299 (1997)) et ce qui est également très dangereux à l'échelle industrielle.

Ainsi, il existe un réel besoin de développer un nouveau procédé de synthèse de la 2-thiohistidine et de ses dérivés qui soit applicable au niveau industriel, c'est-à-dire qui ne présente pas de difficultés de purification, qui n'utilise pas de produits ou de solvants dangereux et toxiques pour l'Homme et l'environnement, et qui permet d'accéder au produit, à l'échelle industrielle, avec un bon rendement et un faible coût.

La présente invention a donc pour objet un procédé de synthèse d'un dérivé de 2-thiohistidine de formule (I) suivante : 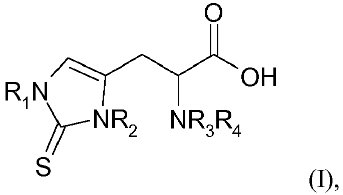 ou d'un sel physiologiquement acceptable de celui-ci, d'un tautomère, d'un stéréoisomère ou d'un mélange de stéréoisomères en toutes proportions, en particulier d'un mélange d'énantiomères, et notamment d'un mélange racémique de celui-ci, pour laquelle :
- R₁ et R₂ représentent, indépendamment l'un de l'autre, un atome d'hydrogène ou un groupe (C₁-C₄)alkyle tel que méthyle, au moins un des groupements R₁ et R₂ représentant un atome d'hydrogène, et avantageusement représentant chacun un atome d'hydrogène, et
- R₃ et R₄ représentent, indépendamment l'un de l'autre, un atome d'hydrogène ou un groupe (C₁-C₄)alkyle tel que méthyle,
comprenant les étapes successives suivantes:
(i) réaction de clivage d'un composé de formule (II) suivante : 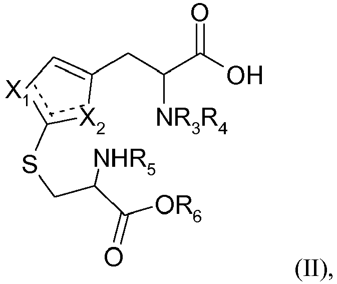 ou d'un sel physiologiquement acceptable de celui-ci, d'un tautomère, d'un stéréoisomère ou d'un mélange de stéréoisomères en toutes proportions, en particulier d'un mélange d'énantiomères, et notamment d'un mélange racémique de celui-ci,
   pour laquelle :
   - 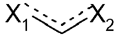 représente 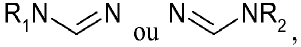
   - R₁, R₂, R₃ et R₄ sont tels que définis ci-dessus,
   - R₅ représente un atome d'hydrogène ou un groupe (C₁-C₄)alkyle ou -CO-((C₁-C₄)alkyle), et en particulier un atome d'hydrogène ou un groupe -**COCH₃**, et plus particulièrement un atome d'hydrogène, et
   - R₆ représente un atome d'hydrogène ou un groupe (C₁-C₄)alkyle, et en particulier un atome d'hydrogène,
   en présence d'un thiol, de préférence soluble dans le solvant de réaction qui pourra être notamment de l'eau, à une température supérieure ou égale à 60°C, pour donner un composé de formule (I), et
(ii) séparation du composé de formule (I) obtenu à l'étape (i) précédente du milieu réactionnel.

Par « tautomère », on entend, au sens de la présente invention, un isomère de constitution du composé obtenu par prototropie, c'est-à-dire par migration d'un atome d'hydrogène et changement de localisation d'une double liaison. Les différents tautomères d'un composé sont généralement interconvertibles et présents en équilibre en solution, dans des proportions qui peuvent varier selon le solvant utilisé, la température ou encore le pH.

Dans le cadre des composés de l'invention, le cycle 2-thioimidazole peut être présent sous les différentes formes tautomères suivantes :

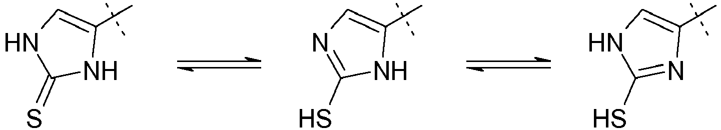

Dans la présente invention, on entend désigner par « physiologiquement acceptable » ce qui est généralement sûr, non toxique et ni biologiquement ni autrement non souhaitable et qui est acceptable pour une utilisation pharmaceutique, cosmétique ou alimentaire (humaine ou animale).

On entend désigner par « sels physiologiquement acceptables » d'un composé, des sels qui sont physiologiquement acceptables, comme défini ci-dessus, et qui possèdent l'activité (pharmacologique, cosmétique ou alimentaire) souhaitée du composé parent. De tels sels comprennent :
(1) les hydrates et les solvates,
(2) les sels d'addition d'acide formés avec des acides inorganiques tels que l'acide chlorhydrique, l'acide bromhydrique, l'acide sulfurique, l'acide nitrique, l'acide phosphorique et similaires ; ou formés avec des acides organiques tels que l'acide acétique, l'acide benzènesulfonique, l'acide benzoïque, l'acide camphresulfonique, l'acide citrique, l'acide éthanesulfonique, l'acide fumarique, l'acide glucoheptonique, l'acide gluconique, l'acide glutamique, l'acide glycolique, l'acide hydroxynaphtoïque, l'acide 2-hydroxyéthanesulfonique, l'acide lactique, l'acide maléique, l'acide malique, l'acide mandélique, l'acide méthanesulfonique, l'acide muconique, l'acide 2-naphtalènesulfonique, l'acide propionique, l'acide salicylique, l'acide succinique, l'acide dibenzoyl-L-tartrique, l'acide tartrique, l'acide p-toluènesulfonique, l'acide triméthylacétique, l'acide trifluoroacétique et similaires, ou
(3) les sels formés lorsqu'un proton acide présent dans le composé parent est soit remplacé par un ion métallique, par exemple un ion de métal alcalin, un ion de métal alcalino-terreux ou un ion d'aluminium ; soit coordonné avec une base organique ou inorganique. Les bases organiques acceptables comprennent la diéthanolamine, l'éthanolamine, N-méthylglucamine, la triéthanolamine, la trométhamine et similaires. Les bases inorganiques acceptables comprennent l'hydroxyde d'aluminium, l'hydroxyde de calcium, l'hydroxyde de potassium, le carbonate de sodium et l'hydroxyde de sodium.

Par « stéréoisomères », on entend, au sens de la présente invention, des diastéréoisomères et des énantiomères. Il s'agit donc d'isomères optiques. Les stéréoisomères qui ne sont pas des images dans un miroir l'un de l'autre sont désignés par « diastéréoisomères », et les stéréoisomères qui sont des images dans un miroir l'un de l'autre, mais non superposables, sont désignés par « énantiomères ».

Un mélange contenant des quantités égales de deux formes énantiomères individuelles de chiralité opposée est désigné par « mélange racémique ».

Par groupement « (C₁-C₄)alkyle », on entend, au sens de la présente invention, une chaîne hydrocarbonée saturée, linéaire ou ramifiée, comportant 1 à 4 atomes de carbone. Il pourra s'agir des groupes méthyle, éthyle, propyle, isopropyle, butyle, isobutyle, sec-butyle, ou tert-butyle. En particulier il s'agira du groupe méthyle.

Par « thiol », on entend, au sens de la présente invention, tout réactif contenant un groupement SH dans sa structure moléculaire. Il s'agira plus particulièrement d'un composé de formule R-SH avec R représentant une chaine hydrocarbonée saturée en C₁ à C₈, notamment en C₂ à C₆, linéaire ou ramifiée, substituée par un ou plusieurs substituants polaires.

Par « chaîne hydrocarbonée saturée », on entend, au sens de la présente invention, une chaîne hydrocarbonée saturée, linéaire ou ramifiée, comprenant avantageusement 1 à 8 atomes de carbone. Il pourra s'agir plus particulièrement d'une chaîne saturée, linéaire, telle qu'un groupe méthyle, éthyle, propyle, butyle, pentyle ou encore hexyle.

Par substituants polaires, on entend, au sens de la présente invention, des groupements hydrophiles tels que les groupements OH, SH, NH₂ et COOH.

Par « réaction de clivage », on entend, au sens de la présente invention, que le composé engagé dans cette réaction est scindé en deux parties lors de cette réaction pour permettre de former la fonction thiocarbonyle du composé de formule (I).

Le composé de formule (I) pourra être en particulier un composé de formule (Ia) suivante : 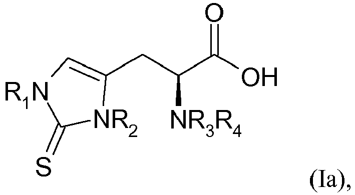 ou un sel physiologiquement acceptable, un tautomère, un stéréoisomère ou un mélange de stéréoisomères en toutes proportions, en particulier un mélange d'énantiomères, et notamment un mélange racémique de celui-ci,
pour laquelle R₁, R₂, R₃ et R₄ sont tels que définis précédemment.

Le composé de formule (I) pourra être notamment la 2-thiohistidine (notamment sous forme D, L ou un mélange racémique D,L), l'α-méthyl-2-thiohistidine et l'α,α-diméthyl-2-thiohistidine, et sera en particulier la 2-thiohistidine et notamment la L-2-thiohistidine.

### Etape (i) :

Cette réaction de clivage réalisée en présence d'un thiol permet d'obtenir le composé de formule (I) ainsi que de l'acide pyruvique (CH₃C(O)-CO₂H) ou un de ses dérivés, notamment un ester (CH₃C(O)-CO₂R₆) ou un dérivé obtenu par réaction avec le thiol, tel qu'un dérivé thiocétalique (deux molécules de thiol pouvant réagir avec la fonction cétone de l'acide pyruvique).

Au contraire, la réaction d'Ito correspond à une hydrolyse réductrice donnant la 2-thiohistidine et de la (D,L)-alanine et non de l'acide pyruvique. Ce résultat pourrait s'expliquer par le fait que la réaction de clivage est effectuée en présence de HI et de phosphore rouge qui génère *in situ* de l'hydrogène (J. Organomet. Chem. 529, 295-299 (1997)).

L'obtention de l'acide pyruvique ou d'un de ses dérivés à la place de la (D,L)-alanine (comme obtenue dans le procédé d'Ito, J. Org. Chem. 1985, 50, 3636-3638) lors de cette réaction de clivage présente l'avantage de faciliter la purification ultérieure du composé de formule (I) puisque l'acide pyruvique (ou ses dérivés) est soluble dans des solvants organiques tandis que la (D,L)-alanine est soluble dans l'eau tout comme le sont les composés de formule (I). En outre, la formation d'hydrogène au cours de cette réaction est fortement déconseillée à l'échelle industrielle en raison de son caractère explosif.

Par ailleurs, le thiol devra être de préférence soluble dans le solvant de réaction qui pourra être notamment de l'eau, ce qui présente l'avantage supplémentaire d'être plus écologique.

Le thiol utilisé dans cette étape (i) pourra être plus particulièrement un thiol répondant à la formule R-SH, avec R représentant une chaîne alkyle, linéaire ou ramifiée, et de préférence linéaire, comportant de 1 à 8, notamment 2 à 6, en particulier 2 à 4, atomes de carbone, substituée par un ou plusieurs groupes choisis parmi OH, SH, NH₂ et COOH.

La présence de groupements hydrophiles (OH, SH, NH₂ et COOH) pourra permettre notamment de rendre le thiol plus soluble dans l'eau, lorsque l'eau est utilisée comme solvant.

Le thiol pourra être choisi parmi la cystéine, le dithiothréitol, le 2-mercaptoéthanol, l'acide 2-mercaptopropionique, l'acide 3-mercaptopropionique et l'acide thioglycolique, et de préférence sera l'acide 3-mercaptopropionique.

Il pourra s'agir également de l'acide mercaptoacétique et l'acide mercaptohexanoïque.

Avantageusement, on utilisera au moins 2 équivalents molaires de thiol par rapport au composé (II), c'est-à-dire qu'au moins 2 moles de thiol sont utilisées pour une mole de composé (II) utilisée. On pourra en particulier utiliser au moins 5 équivalents molaires de thiol, et notamment 5 à 10 équivalents molaires de thiol, par rapport au composé (II).

Le mélange réactionnel est chauffé à une température supérieure à 60°C car en-dessous de cette température, la cinétique de la réaction serait trop lente. La réaction pourra être réalisée à une température comprise entre 60 et 120°C, notamment entre 80 et 100°C, plus particulièrement après addition du thiol.

La réaction pourra être réalisée notamment en milieu acide.

### Etape (ii) :

Le produit final obtenu (composé de formule (I)) pourra être séparé du milieu réactionnel par des techniques bien connues de l'homme du métier et applicables à l'échelle industrielle, en particulier par précipitation du composé de formule (I) notamment en ajustant le pH de la solution pour arriver par exemple à un pH compris entre 5,5 et 6,5, de préférence d'environ 6 (plus particulièrement dans le cas de la 2-thiohistidine) ou par évaporation, éventuellement partielle, des solvants suivie de préférence d'une recristallisation pour purifier le produit.

Les composés de formule (I) étant solubles dans l'eau, une ou plusieurs extractions préalables avec un solvant organique, tel que l'acétate d'éthyle ou l'éther ter-butyl-méthylique, pourront permettre d'éliminer les sous-produits organiques formés au cours de la réaction, tel que l'acide pyruvique ou ses dérivés, ainsi que l'excès de thiol.

Le produit obtenu pourra être purifié si nécessaire par des techniques bien connues de l'homme du métier, par exemple par recristallisation.

Avant ou après cette étape (ii), un sel du composé formé pourra être préparé, si cela est souhaité, notamment par addition d'un acide ou d'une base physiologiquement acceptable tels que définis précédemment.

Le composé de formule (II) pourra être préparé à partir d'un sel d'addition d'acide, à l'exception d'un sel de l'acide iodhydrique (HI), du composé de formule (III) suivante : 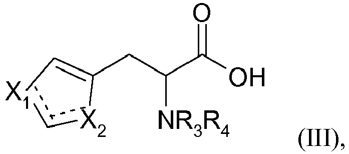 ou d'un tautomère ou d'un stéréoisomère ou d'un mélange de stéréoisomères en toutes proportions, en particulier d'un mélange d'énantiomères, et notamment d'un mélange racémique de celui-ci,
pour laquelle 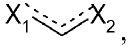 R₃ et R₄ sont tels que définis précédemment,
par réaction successivement avec du dibrome,
puis avec un dérivé de cystéine de formule (IV) suivante : 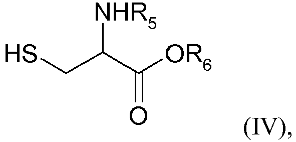 ou un stéréoisomère ou un mélange de stéréoisomères en toutes proportions, en particulier un mélange d'énantiomères, et notamment un mélange racémique de celui-ci, dans laquelle R₅ et R₆ sont tels que définis précédemment.

Par « sel d'addition d'acide du composé de formule (III) », on entend, au sens de la présente invention, un sel du composé de formule (III) obtenu par addition d'un acide, à l'exclusion de l'acide iodhydrique HI. L'acide pourra être en particulier de l'acide chlorhydrique ou de l'acide sulfurique.

Dans cette réaction, le dibrome pourra être utilisé à raison de 1 à 1,5 équivalents molaires par rapport au composé de formule (III).

De préférence, le dibrome est ajouté à froid (addition très rapide de préférence), à une température inférieure à 10°C, de préférence inférieure à 5°C. L'addition du dibrome pourra donc être réalisée à une température comprise entre -10°C et 10°C, avantageusement comprise entre -5°C et 5°C.

Le dérivé de cystéine pourra être en particulier la N-acétylcystéine ou la cystéine (notamment sous forme D, L ou racémique), et en particulier de la cystéine et notamment de la L-cystéine.

Le dérivé de cystéine sera avantageusement utilisé en excès, en particulier à raison de 2 à 7, avantageusement 3 à 5 équivalents molaires de dérivé de cystéine par rapport au composé de formule (III), c'est-à-dire que de 2 à 7, avantageusement 3 à 5 moles de dérivé de cystéine sont utilisées pour une mole de composé (III) utilisée.

Cette réaction pourra être réalisée dans un solvant tel que l'eau.

Le rendement de cette étape pourra être supérieur ou égal à 50%, voire supérieur ou égal à 70%.

De préférence, le composé de formule (II) ne sera pas isolé du milieu réactionnel mais sera engagé directement dans l'étape (i) suivante. Ainsi, la préparation du composé (I) à partir du composé (III) peut être réalisée dans un seul réacteur, sans isolement du composé (II) intermédiaire (réaction « one-pot »).

Le procédé de préparation d'un composé de formule (I) selon l'invention pourra donc comprendre les étapes successives suivantes :
(**a1**) réaction d'un sel d'addition d'acide, à l'exclusion du sel de l'acide iodhydrique, d'un composé de formule (III) tel que défini ci-dessus, ou d'un tautomère, d'un stéréoisomère ou d'un mélange de stéréoisomères en toutes proportions, en particulier d'un mélange d'énantiomères, et notamment d'un mélange racémique de celui-ci, avec du dibrome,
   puis avec un dérivé de cystéine de formule (IV) tel que défini ci-dessus ou un stéréoisomère ou un mélange de stéréoisomères en toutes proportions, en particulier un mélange d'énantiomères, et notamment un mélange racémique de celui-ci, et en particulier avec de la cystéine et notamment de la L-cystéine,
   pour donner une composé de formule (II) tel que défini ci-dessus,
(**b1**) réaction de clivage du composé de formule (II) obtenu à l'étape (a1) précédente en présence d'un thiol tel que défini précédemment, de préférence soluble dans le solvant de réaction qui pourra être notamment de l'eau, à une température supérieure ou égale à 60°C, pour donner un composé de formule (I), et
(**c1**) séparation du composé de formule (I) obtenu à l'étape (b1) précédente du milieu réactionnel.

Les étapes (b1) et (c1) correspondent respectivement aux étapes (i) et (ii) précédentes. L'étape (a1), quant à elle, correspond à l'étape de préparation du composé de formule (II) décrite précédemment.

Avantageusement, les étapes (a1) et (b1) seront réalisées dans un même solvant, tel que l'eau, de préférence, dans un même réacteur, c'est-à-dire sans isolement des produits intermédiaires (composé de formule (II) en particulier).

Dans ces conditions, le milieu réactionnel pourra contenir un dérivé de cystéine utilisé de préférence en excès à l'étape (a1). Avant de séparer le composé de formule (I) du milieu réactionnel (étape (c1)), il pourra donc être nécessaire d'éliminer l'excès de dérivé de cystéine afin de faciliter l'isolement et la purification du composé de formule (I). Notamment, dans le cas d'un dérivé de cystéine pour lequel R₅ = H ou (C₁-C₄)alkyle tel que la cystéine, il peut être ajouté par exemple du benzaldéhyde qui formera alors avec le dérivé de cystéine en excès un dérivé de l'acide 2-phénylthiazolidine-4-carboxylique, composé qui précipite dans un solvant tel que l'eau. Par ce moyen, le dérivé de cystéine en excès pourrait être recyclé.

Le rendement global de préparation du composé de formule (I) à partir du composé de formule (III) pourra être supérieur ou égal à 40%.

Selon un mode de réalisation particulier de l'invention, le composé de formule (I) est un composé de formule (Ia) et son procédé de préparation comprend les étapes successives suivantes :
(**a2**) réaction d'un sel d'addition d'acide, à l'exclusion du sel de l'acide iodhydrique, d'un composé de formule (IIIa) suivante : 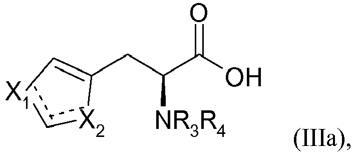 ou d'un tautomère ou d'un stéréoisomère ou d'un mélange de stéréoisomères en toutes proportions, en particulier d'un mélange d'énantiomères, et notamment d'un mélange racémique de celui-ci,
   pour laquelle 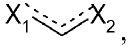 R₃ et R₄ sont tels que définis précédemment, avec du dibrome,
   puis avec un dérivé de cystéine de formule (IV) tel que défini ci-dessus ou un stéréoisomère ou un mélange de stéréoisomères en toutes proportions, en particulier un mélange d'énantiomères, et notamment un mélange racémique de celui-ci, et en particulier avec de la cystéine et notamment de la L-cystéine,
   pour donner un composé de formule (IIa) suivante : 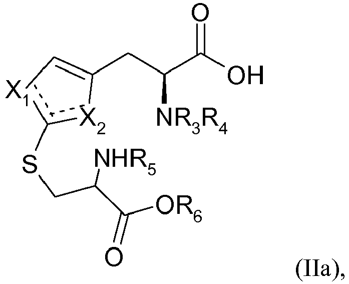 ou un sel physiologiquement acceptable, un tautomère, un stéréoisomère ou un mélange de stéréoisomères en toutes proportions, en particulier un mélange d'énantiomères, et notamment un mélange racémique de celui-ci,
   pour laquelle 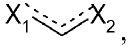 R₃, R₄, R₅ et R₆ sont tels que définis précédemment,
(**b2**) réaction de clivage du composé de formule (IIa) obtenu à l'étape (a2) précédente en présence d'un thiol tel que défini précédemment, de préférence soluble dans le solvant de réaction qui pourra être de l'eau, et en particulier avec la cystéine, le dithiothréitol, le 2-mercaptoéthanol, l'acide 2-mercaptopropionique, l'acide 3-mercaptopropionique ou l'acide thioglycolique, et de préférence avec l'acide 3-mercaptopropionique, à une température supérieure ou égale à 60°C,
   pour donner un composé de formule (Ia), et
(**c2**) séparation du composé de formule (Ia) obtenu à l'étape (b2) précédente du milieu réactionnel.

Les étapes (a2), (b2) et (c2) correspondent respectivement aux étapes (a1), (b1) et (c1) précédentes.

Les composés de formule (IIa) représentent des formes particulières du composé de formule (II). De même, les composés de formule (IIIa) représentent des formes particulières du composé de formule (III).

Un composé de formule (II) suivante est également décrit : 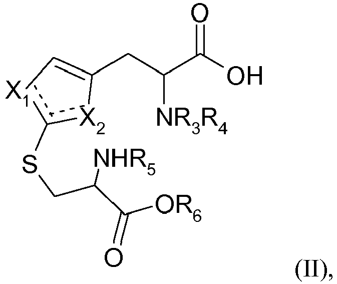 ou un sel physiologiquement acceptable, un tautomère, un stéréoisomère ou un mélange de stéréoisomères en toutes proportions, en particulier un mélange d'énantiomères, et notamment un mélange racémique de celui-ci, pour laquelle :
- 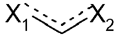 représente 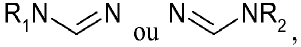 et
- R₁, R₂, R₃, R₄, R₅ et R₆ sont tels que définis précédemment, à l'exclusion du composé pour lequel 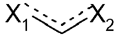 représente 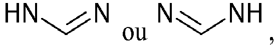 et R₃ et R₄ représentent chacun un atome d'hydrogène.

Le composé exclu est décrit dans : Ito et al. J. Org. Chem. 1985, 50, 3636-3638.

Ce composé pourra être en particulier le 2-{2-[(2-ammonio-2-carboxyéthyl)thio]-1*H*-imidazol-4-yl}-1-carboxy-*N*,*N*-diméthyléthanaminium (HisNMe₂-Cys) ou son dichlorhydrate, et le 2-{2-[(2-ammonio-2-carboxyéthyl)thio]-1*H*-imidazol-4-yl}-1-carboxy-*N*-méthyléthanaminium (HisNHMe-Cys) ou son dichlorhydrate.

Un procédé de préparation d'un composé de formule (II) suivante est également décrit : 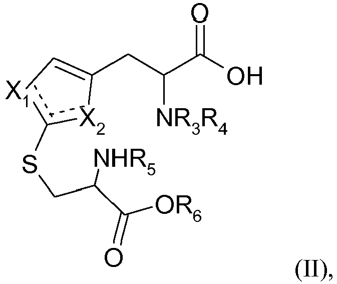 ou un sel physiologiquement acceptable, un tautomère, un stéréoisomère ou un mélange de stéréoisomères en toutes proportions, en particulier un mélange d'énantiomères, et notamment un mélange racémique de celui-ci, pour laquelle :
- 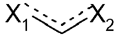 représente 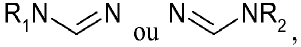 et
- R₁, R₂, R₃, R₄, R₅ et R₆ sont tels que définis précédemment,
par réaction d'un sel d'addition d'acide, à l'exclusion du sel de l'acide iodhydrique, d'un composé de formule (III) tel que défini précédemment, ou d'un tautomère d'un stéréoisomère ou d'un mélange de stéréoisomères en toutes proportions, en particulier d'un mélange d'énantiomères, et notamment d'un mélange racémique de celui-ci,
avec du dibrome, puis avec 2 à 7, de préférence 3 à 5 équivalents molaires, par rapport au composé de formule (III), d'un dérivé de cystéine de formule (IV) tel que défini précédemment, et en particulier de la cystéine et notamment de la L-cystéine,.

Dans cette réaction, le dibrome pourra être utilisé à raison de 1 à 1,5 équivalents molaires par rapport au composé de formule (III).

De préférence, le dibrome est ajouté à froid (addition très rapide de préférence), à une température inférieure à 10°C, de préférence inférieure à 5°C. L'addition du dibrome pourra donc être réalisée à une température comprise entre -10°C et 10°C, avantageusement comprise entre -5°C et 5°C.

Cette réaction pourra être réalisée dans un solvant tel que l'eau.

La présente invention sera mieux comprise à la lumière des exemples qui suivent, qui sont fournis simplement à titre d'illustration et ne sauraient en aucune façon limiter la portée de l'invention.

### EXEMPLES

Toutes les réactions ont été réalisées à l'air libre sauf indication contraire.

### 1-Préparation des composés de formule (II)

### Exemple 1 : Préparation du dichlorhydrate du 2-{2-[(2-ammonio-2-carboxyéthyl)thio]-1H-imidazol-4-yl}-1-carboxy-éthanaminium (His-Cys, 2HCl)

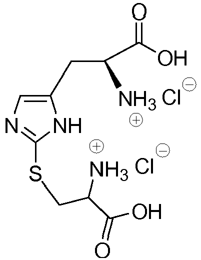

1,57 g (7,5 mmoles) du chlorhydrate monohydraté de la L-Histidine sont dissous dans 15 mL d'eau, et on refroidit la solution à 0°C. Sous très forte agitation, 500 µL (1,56 g, 9,75 mmoles, 1,3 équiv.) de dibrome sont ajoutés goutte à goutte (temps d'addition 2min10). Le mélange réactionnel se colore en jaune. Trois minutes après la fin de l'addition du dibrome, 2,81 g (22,5 mmoles, 3 équiv.) de L-Cystéine sont ajoutés. Immédiatement, le mélange se décolore. Après agitation à 0°C pendant 1h, le mélange est filtré, et le précipité lavé avec 2x0,5 mL d'eau.
Le filtrat est déposé sur une colonne remplie avec 75g de DOWEX^{®} 50WX2-400, conditionnée auparavant avec de l'acide chlorhydrique HCl 1N. Après élution avec 750 mL d'acide chlorhydrique HCl 1N, puis 500 mL d'acide chlorhydrique HCl 2N, les fractions contenant le produit désiré sont réunies. Après évaporation et 2 co-évaporations avec 2x20 mL de toluène, on obtient après séchage 1,5 g (56%) du produit désiré sous forme de cristaux jaunes.
¹H-RMN (D₂O/DCl, 400 MHz) : δ (ppm) = 3,32 (m, 2H) ; 3,63 (m, 2H) ; 4,22 (m, 2H) ; 7,39 (s, 1H).
UPLC-MS (ES+) : 275,8 (MH+)

### Exemple 2 : Préparation du dichlorhydrate du 2-{2-[(2-ammonio-2-carboxyéthyl)thio]-1H-imidazol-4-yl}-1-carboxy-N,N-diméthyléthanaminium (HisNMe₂-Cys, 2HCl)

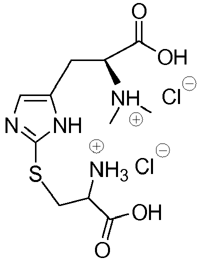

4,753 g (20 mmoles) de chlorhydrtae monohydraté de N,N-diméthylhistidine (V. N. Reinhold et al., J. Med. Chem. 11, 258 (1968*)*) sont dissous dans 40 mL d'eau, et la solution est refroidie à 0°C. Sous très forte agitation, 1,23 mL (3,835g, 24 mmoles, 1,2 équiv.) de dibrome sont ajoutés goutte à goutte rapidement (temps d'addition 1min20) sans dépasser 2°C. Le mélange réactionnel se colore en jaune, et un solide rougeâtre se forme. Sept minutes après la fin de l'addition du dibrome, tout le solide rouge s'est dissout, et on ajoute 7,417 g (60 mmoles, 3 équiv.) de L-Cystéine. Immédiatement, le mélange se décolore. Après agitation à 0°C pendant 1h, on obtient une suspension blanche. Le mélange est filtré, et le précipité lavé avec 2x2 mL d'eau.
Le filtrat est déposé sur une colonne remplie avec 100g de DOWEX^{®} 50WX2-400, conditionnée auparavant avec de l'acide chlorhydrique HCl 1N. Après élution avec 800 mL d'acide chlorhydrique HCl 1N, puis 1000 mL d'acide chlorhydrique HCl 2N, les fractions contenant le produit désiré sont réunies. Après évaporation et 2 co-évaporations avec 2x50 mL de toluène, on obtient après séchage 4,84 g (63%) du produit désiré sous forme de cristaux légèrement jaunes.
¹H-RMN (D₂O, 400 MHz) : δ (ppm) = 2,86 (s, 6H) ; 3,32 (m, 2H) ; 3,61 (m, 2H) ; 4,21 (m, 1H) ; 4,32 (m, 1H) ; 7,37 (s, 1H).
UPLC-MS (ES+) : 303,8 (MH+)

### Exemple 3 : Préparation du dichlorhydrate du 2-{2-[(2-ammonio-2-carboxyéthyl)thio]-1H-imidazol-4-yl}-1-carboxy-N-méthyléthanaminium (HisNHMe-Cys, 2HCl)

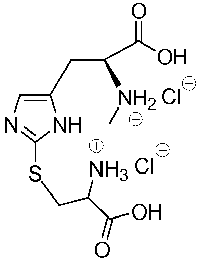

1,187 g (5,656 mmoles) de chlorhydrate de α-N-méthylhistidine (V. N. Reinhold et al., J. Med. Chem. 11, 258 (1968*)*) sont dissous dans 11,3 mL d'eau déminéralisée. La solution est refroidie à 0°C et 377 µL (1,175 g, 7,353 mmoles, 1,3 équiv.) de dibrome sont ajoutés goutte à goutte sous très forte agitation (temps d'addition 1min45). Le mélange réactionnel se colore en jaune, et la température monte à 4°C.
Trois minutes après la fin de l'addition du dibrome, 2,119 g (16,96 mmoles, 3 équiv.) de L-Cystéine sont ajoutés. Immédiatement, le mélange se décolore. Après agitation à 0°C pendant 1h, le mélange est déposé sur une colonne remplie avec 50g de DOWEX^{®} 50WX2-400, conditionnée auparavant avec de l'acide chlorhydrique 0,5N. Après élution avec 250 mL d'acide chlorhydrique 0,5N, puis 250 mL d'acide chlorhydrique 1N et 500 mL d'acide chlorhydrique 1,5N, les fractions contenant le produit désiré sont réunies. Après évaporation et séchage sous vide (20 mbar), on obtient 1,427 g (68%) du produit désiré sous forme de cristaux jaunes.
¹H-RMN (D₂O, 400 MHz) : δ (ppm) = 2,71 (s, 3H) ; 3,33 (m, 2H) ; 3,62 (m, 2H) ; 4,08 (m, 1H) ; 4,28 (m, 1H) ; 7,40 (s, 1H).
UPLC-MS (ES+) : 289,8 (MH+)

### 2- Préparation des composés de formule (I) selon l'invention (sans isolement des intermédiaires de formule (II))

### 2-1- Préparation de la 2-thiohistidine

### Exemple 4 : Préparation de la L-2-Thiohistidine à partir de l'Histidine (« one-pot »)

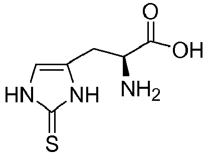

### a) Synthèse de l'intermédiaire His-Cys (composé de formule (II))

317,6 g (1,5 moles) du chlorhydrate monohydraté de la L-Histidine sont dissous dans 3 L d'eau déminéralisée. La solution est transférée dans un réacteur en verre double enveloppe avec agitation mécanique et est refroidie à -4°C. Sous très forte agitation, 100,2 mL (311,6 g, 1,95 moles, 1,3 équiv.) de dibrome sont ajoutés très rapidement via une ampoule à brome, sans dépasser 4°C (temps d'addition 2min50). Le mélange réactionnel prend une coloration jaune-orange, mais reste clair et homogène. 3 min. après la fin de l'addition du dibrome, 562 g (4,5 moles, 3 équiv.) de L-Cystéine sont ajoutés, et la température interne monte à 12°C. Immédiatement, le mélange se décolore. Après quelques minutes, la solution devient légèrement jaune.
Après agitation à 0°C pendant 1h, une analyse d'un échantillon par ¹H-RMN (D₂O) montre que l'adduit His-Cys est formé avec un rendement réactionnel de 70 %.
Le système de refroidissement est arrêté, et on laisse agiter le mélange réactionnel pendant une heure. La température interne monte à 12°C. L'adduit His-Cys n'est pas isolé et est engagé directement dans l'étape suivante.

### b) Synthèse de la L-2-Thiohistidine

On ajoute ensuite 793,4 mL (960 g, 9 moles, 6 équiv.) d'acide 3-mercaptopropionique au mélange, et l'on chauffe sous forte agitation à 100°C pour 18h.
Une analyse d'un échantillon par ¹H-RMN (D₂O) montre que l'adduit His-Cys est complètement clivé en L-2-Thiohistidine.

### c) Isolement de la L-2-Thiohistidine

Après refroidissement à température ambiante, le mélange d'une couleur marron foncé est extrait avec 4x3 L d'acétate d'éthyle.
La phase aqueuse est retenue, et placé sous un fort courant d'azote dans un bain à 40°C. Le pH du mélange est ajusté à 8,5 en ajoutant rapidement une solution d'ammoniaque aqueuse à 20% (environ 580 mL). Toujours sous azote, on ajoute ensuite goutte à goutte de l'acide chlorhydrique concentré à 37% (environ 120 mL), jusqu'à un pH de 6,5. Un précipité incolore se forme au cours de l'addition de l'acide, et on maintient la suspension pendant une heure à 40°C sous agitation (sous azote).
Après filtration et lavage avec 3x300 mL d'eau déminéralisée, suivi par 2 lavages avec 600mL d'éthanol absolue et 3x600mL de n-pentane, le solide obtenu est séché sur P₂O₅ sous vide (20 mbar). On obtient 130,1 g (45%) de la L-2-Thiohistidine sous forme de poudre blanche.
Les données analytiques du produit obtenu sont identiques à celles de la littérature (J. Xu, J. C. Yadan, J. Org. Chem. 60, 6296 - 6301 (1995*)*).
[α]_{D} : -11,0 (c = 1,0 ; 1N HCl)
¹H-RMN (D₂O/DCl, 400 MHz) : δ (ppm) = 3,10 (m, 2H) ; 4,20 (m, 1H) ; 6,77 (s, 1H).
UPLC-MS (ES+) : 188,6 (MH+)

Les exemples 5 à 8 suivant montrent la variabilité du sel d'addition d'acide du composé de formule (III) qui peut être utilisé ainsi que la variabilité du thiol qui peut être utilisé. Ces exemples sont uniquement illustratifs et ne servent en aucune façon à limiter la portée de la présente invention.

### Exemple 5 : Préparation de la L-2-Thiohistidine à partir de la L-Histidine (« one-pot ») - variation du sel d'addition d'acide

On procède comme décrit dans l'Exemple 2, sauf que l'on utilise 5 g (32,2 mmoles) de la L-Histidine, dissout dans 65 mL d'eau et 900 µL (1,66g, 16,1 mmoles, 1 équiv.) de l'acide sulfurique à 98%.
On obtient 2,72 g (45 %) de la L-2-Thiohistidine sous forme de poudre blanche. L'analyse ¹H-RMN (D₂O/DCl) est identique à celle décrite dans l'Exemple 1.

### Exemple 6 : Préparation de la L-2-Thiohistidine à partir de la L-Histidine (« one-pot ») - variation du thiol (acide mercaptoacétique)

3,5 g (16,52 mmoles) du chlorhydrate monohydraté de la L-Histidine sont dissous dans 33 mL d'eau, et la solution est refroidie à -3°C. Sous très forte agitation, 1,1 mL (3,433 g, 21,48 mmoles, 1,3 équiv.) de dibrome sont ajoutés goutte à goutte rapidement (temps d'addition 3min). Le mélange réactionnel se colore en jaune. Trois minutes après la fin d'addition du dibrome, 6,19 g (49,58 mmoles, 3 équiv.) de L-Cystéine sont ajoutés. Immédiatement, le mélange se décolore. Après agitation à 0°C pendant 30 min., on ajoute 7,13 mL (9,4g, 99,17 mmoles) d'acide mercaptoacétique, puis on chauffe en agitant à 80°C pendant 40h.
Pour l'isolement de la L-2-Thiohistidine, on procède comme décrit dans l'Exemple 1. On obtient 43 % de la L-2-Thiohistidine sous forme de poudre de couleur blanc crème. L'analyse ¹H-RMN (D₂O/DCl) est identique à celle décrite dans l'Exemple 1.

### Exemple 7 : Préparation de la L-2-Thiohistidine à partir de la L-Histidine (« one-pot ») - variation du thiol (acide mercaptohexanoïque)

257 mg (1,21 mmoles) du chlorhydrate monohydraté de la L-Histidine sont dissous dans 2,4 mL d'eau, et la solution est refroidie à -3°C. Sous très forte agitation, 80 µL (252 mg, 1,58 mmoles, 1,3 équiv.) de dibrome sont ajoutés goutte à goutte rapidement. Le mélange réactionnel se colore en jaune. Trois minutes après la fin de l'addition du dibrome, 455 mg (3,64 mmoles, 3 équiv.) de L-Cystéine sont ajoutés. Immédiatement, le mélange se décolore. Après agitation à 0°C pendant 1h, on ajoute 933 µL (1,0 g, 6,07 mmoles) d'acide mercaptohexanoique, puis on chauffe en agitant à 80°C pendant 40h.
Une analyse d'un échantillon par ¹H-RMN (D₂O) montre que l'adduit His-Cys est complètement clivé en L-2-Thiohistidine.

### Exemple 8 : Préparation de la L-2-Thiohistidine à partir de la L-Histidine (« one-pot ») - variation du thiol (dithiotréitol)

10,72 g (50,62 mmoles) du chlorhydrate monohydraté de la L-Histidine sont dissous dans 100 mL d'eau, et la solution est refroidie à -0°C. Sous très forte agitation, 3,38 mL (10,51 g, 65,81 mmoles, 1,3 équiv.) de dibrome sont ajoutés goutte à goutte rapidement (temps d'addition 3min20), sans dépasser 1°C. Le mélange réactionnel se colore en jaune. Trois minutes après la fin de l'addition du dibrome, 18,96 g (151,8 mmoles, 3 équiv.) de L-Cystéine sont ajoutés. Immédiatement, le mélange se décolore. Après agitation à 0°C pendant 1h, on ajoute 47,08g (303,7 mmoles, 6 équiv.) de Dithiothréitol, puis on chauffe en agitant à 80°C pendant 40h.
Pour l'isolement de la L-2-Thiohistidine, on procède comme décrit dans l'Exemple 1. On obtient 41 % de la L-2-Thiohistidine sous forme de poudre blanche. L'analyse ¹H-RMN (D₂O/DCl) est identique à celle décrite dans l'Exemple 1.

### Exemple 9 : Préparation de la D-2-Thiohistidine à partir de la D-Histidine (« one-pot »)

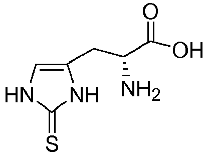

On procède comme décrit dans l'Exemple 2-1, sauf que l'on utilise 10,32 g (65,84 mmoles) de la D-Histidine, dissout dans 132 mL d'eau et 5,5 mL (6,48g, 65,84 mmoles) d'acide chlorhydrique concentré.
On obtient 4,4 g (35 %) de la D-2-Thiohistidine sous forme de poudre blanche. L'analyse ¹H-RMN (D₂O/DCl) est identique à celle décrite dans l'Exemple 1.

### [α]_{D} : + 10,5 (c = 1,0 ; 1N HCl)

### Exemple 10 : Préparation de la D,L-2-Thiohistidine à partir de la D,L-Histidine (« one-pot »)

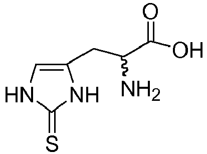

### a) Synthèse de l'adduit His-Cys

10,65 g (50,8 mmoles) du chlorhydrate monohydraté de la D,L-Histidine sont dissous dans 100 mL d'eau déminéralisée. La solution est refroidie à -3°C. Sous très forte agitation, 3,43 mL (10,66 g, 66 mmoles, 1,3 équiv.) de dibrome sont ajoutés très rapidement via une ampoule à brome, sans dépasser 1°C (temps d'addition 2min40). Le mélange réactionnel prend une coloration jaune-orange, mais reste clair et homogène. Trois minutes après la fin de l'addition du dibrome, 19,03 g (152,4 moles, 3 équiv.) de L-Cystéine sont ajoutés, et la température interne monte à 4°C. Immédiatement, le mélange se décolore. Après quelques minutes, la solution devient légèrement jaune.
Après agitation à 0°C pendant 1h, une analyse d'un échantillon par ¹H-RMN (D₂O) montre que l'adduit His-Cys est formé avec un rendement réactionnel de 72%.
Le bain de refroidissement est enlevé, et on laisse agiter le mélange réactionnel pendant 30 min. La température interne monte à 10°C.

### b) Synthèse de la D,L-2-Thiohistidine

On ajoute ensuite 27 mL (32,68 g, 304,8 moles, 6 équiv.) d'acide 3-mercaptopropionique au mélange, et l'on chauffe sous forte agitation à 80°C pour 30h. Une analyse d'un échantillon par ¹H-RMN (D₂O) montre que l'adduit His-Cys est complètement clivé en 2-Thiohistidine.

### c) Isolement de la D,L-2-Thiohistidine

Après refroidissement à température ambiante, le mélange d'une couleur marron-foncé est extrait avec 4x100mL d'acétate d'éthyle.
La phase aqueuse est retenue et placé sous un fort courant d'azote dans un bain à 40°C. Le pH du mélange est ajusté à 6 en ajoutant rapidement une solution d'ammoniaque aqueuse à 20%. Un précipité incolore se forme, et on maintient la suspension pendant 25min à 40°C sous agitation (sous azote).
Après filtration et lavage avec 3x15 mL d'eau déminéralisée, suivi par 2 lavages avec 15mL d'éthanol absolue et 3x15mL de n-pentane, le solide obtenu est séché sur P₂O₅ sous vide (20 mbar). On obtient 5,2 g de la D,L-2-Thiohistidine sous forme de poudre blanche, contenant environ 10% de la Cystéine.
Le produit est purifié par traitement acido-basique : pour cela, on dissout 585 mg (3,79 mmoles) de Dithiothréitol dans 100 mL d'eau déminéralisée, puis on ajoute le produit obtenu précédemment (5,2 g). La suspension est placée sous azote dans un bain d'eau à 40°C, et on ajoute très lentement goutte à goutte 10 mL (11,84g) d'acide chlorhydrique concentré, jusqu'à dissolution complète du solide. Ensuite on ajuste le pH à 6 par addition lente de 3 mL (2,735g) d'une solution aqueuse d'ammoniaque à 20%. Un précipité incolore fin se forme lentement, et la suspension est maintenue sous agitation pendant une heure.
Après filtration, lavage avec 3x10 mL d'eau déminéralisée, suivi par 3 lavages avec 10mL d'éthanol absolue et 3x10mL de n-pentane, le solide obtenu est séché sur P₂O₅ sous vide (20 mbar). On obtient 3,82 g (39 %) de la D,L-2-Thiohistidine sous forme de poudre blanche.
L'analyse ¹H-RMN (D₂O/DCl) est identique avec celle décrite dans l'Exemple 1.
[α]_{D} : 0 (c = 1,0; 1N HCl)

### 2-2- Préparation de dérivés de la 2-Thiohistidine

### Exemple 11 : Préparation de la α,α-diméthyl-2-thiohistidine (« one-pot ») à partir de la α,α -diméthyl-histidine (« one-pot »)

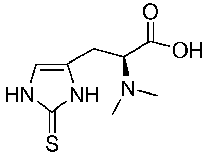

### a) Préparation de l'adduit HisNMe₂-Cys (composé de formule (II))

23,76 g (0,1 mole) de chlorhydrate monohydraté de α,α-diméthylhistidine (V. N. Reinhold et al., J. Med. Chem. 11, 258 (1968*)*) sont dissous dans 200 mL d'eau. La solution est refroidie à -3°C, et sous très forte agitation, 6,68 mL (20,77 g, 130 mmoles, 1,3 équiv.) de dibrome sont ajoutés rapidement goutte à goutte sans dépasser 3°C (temps d'addition 4min). Le mélange réactionnel se colore en jaune, et un solide rougeâtre se forme. Sept minutes après la fin de l'addition du dibrome, le solide rouge s'est complètement dissout, et 37,08 g (0,3 moles, 3 équiv.) de L-Cystéine sont ajoutés. La température interne monte de 0°C à 1°C, et le mélange s'est décoloré immédiatement.
Après agitation à 0°C pendant 1h, une analyse d'un échantillon par ¹H-RMN (D₂O) montre que l'adduit HisNMe₂-Cys est formé avec un rendement réactionnel de 63%. Le bain de glace est enlevé, et on laisse agiter le mélange réactionnel pendant une heure. La température interne monte à 10°C.

### b) Préparation de la α,α-diméthyl-2-thiohistidine

On ajoute ensuite 87,7 mL (106 g, 10 équiv.) d'acide 3-mercaptopropionique au mélange, et l'on chauffe sous forte agitation à 80°C pour 24h.
Une analyse d'un échantillon par ¹H-RMN (D₂O) montre que le produit HisNMe₂-Cys est complètement clivé.

### c) Isolement de la α,α-diméthyl-2-thiohistidine

Après refroidissement à température ambiante, le mélange d'une couleur orange est extrait avec 4x400 mL d'acétate d'éthyle.
La phase aqueuse est retenue, et le pH est ajusté à 4,5 - 5 avec une solution d'ammoniaque aqueuse à 20% (environ 21 mL). Afin de piéger l'excès de la cystéine présente dans le milieu, on ajoute 30,5 mL (31,8 g, 3 équiv.) de benzaldéhyde (selon M. P. Schubert, J. Biol. Chem. 114, 341-350 (1936*) ou* M. Seki et al., J. Org. Chem. 67 (16), 5532 (2002)). Le mélange est agité à température ambiante pendant 15h, et l'acide 2-phénylthiazolidine-4-carboxylique précipite sous forme d'un solide jaune clair. Après filtration du solide et rinçage avec 4x40 mL d'eau, le filtrat est extrait avec 2x200 mL d'acétate d'éthyle.
La phase aqueuse est retenue, et on ajuste le pH à 6 avec une solution d'ammoniaque aqueuse à 20% (environ 2 mL). Le mélange est ensuite évaporé à sec, et le solide obtenu est recristallisé dans l'eau. On obtient 7,28 g (33%) du produit désiré sous forme d'une poudre blanche.
Les données analytiques sont identiques à celles décrites dans la littérature (WO 95/18 108).
¹H-RMN (D₂O/DCl, 400 MHz) : δ (ppm) = 2,87 (s, 6H) ; 3,20 (m, 2H) ; 4,19 (m, 1H) ; 6,78 (s, 1H).
UPLC-MS (ES+) : 216,5 (MH+)

### Exemple 12 : Préparation de α-méthyl-2-thiohistidine à partir de α-méthylhistidine (« one-pot »)

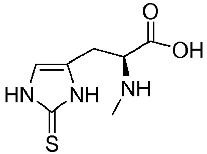

11,93 g (54,5 mmoles) de chlorhydrate de α-méthylhistidine (V. N. Reinhold et al., J. Med. Chem. 11, 258 (1968*)*) sont dissous dans 109 mL d'eau, puis la solution est refroidie à 0°C. Sous très forte agitation, 3,64 mL (11,33 g, 70,9 mmoles, 1,3 équiv.) de dibrome sont ajoutés goutte à goutte sans dépasser 5°C (temps d'addition 2min30). Le mélange réactionnel se colore en jaune. Trois minutes après la fin de l'addition du dibrome, 20,22 g (163,6 mmoles, 3 équiv.) de L-Cystéine sont ajoutés, et la température interne monte à 8°C. Immédiatement, le mélange se décolore. Après agitation à 0°C pendant 1h, on ajoute 28,84 mL (34,9 g, 6 équiv.) d'acide 3-mercaptopropionique au mélange, et l'on chauffe sous forte agitation à 100°C pour 20h.
En poursuivant le traitement en analogie avec l'Exemple 11, on obtient 5,398 g (48%) de α-méthyl-2-thiohistidine sous forme d'une poudre blanche.
¹H-RMN (D₂O, 400 MHz) : δ (ppm) = 2,65 (s, 3H) ; 3,15 (m, 2H) ; 4,12 (m, 1H) ; 6,77 (s, 1H).
¹³C-RMN (D₂O, 400 MHz) : δ (ppm) = 24,8 ; 32,3 ; 60,0 ; 116,4 ; 123,2 ; 156,9 ; 170,0. UPLC-MS (ES+) : 202,7 (MH+)

## Revendications

1. Procédé de synthèse d'un dérivé de 2-thiohistidine de formule (I) suivante : 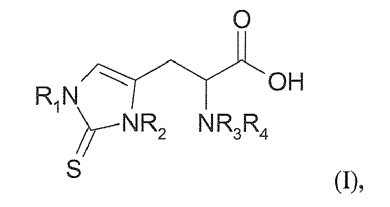 ou d'un sel physiologiquement acceptable de celui-ci, d'un tautomère, d'un stéréoisomère ou d'un mélange de stéréoisomères en toutes proportions,
pour laquelle :
- R₁ et R₂ représentent, indépendamment l'un de l'autre, un atome d'hydrogène ou un groupe (C₁-C₄)alkyle, au moins un des groupements R₁ et R₂ représentant un atome d'hydrogène, et
- R₃ et R₄ représentent, indépendamment l'un de l'autre, un atome d'hydrogène ou un groupe (C₁-C₄)alkyle,
comprenant les étapes successives suivantes :
(i) réaction de clivage d'un composé de formule (II) suivante : 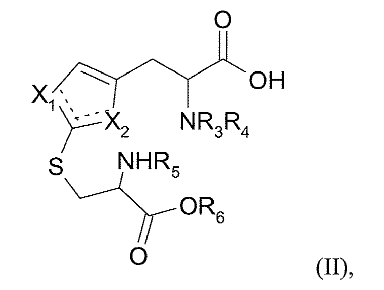 ou d'un sel physiologiquement acceptable, d'un tautomère, d'un stéréoisomère ou d'un mélange de stéréoisomères en toutes proportions, pour laquelle :
- 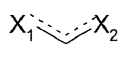 représente 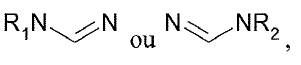
- R₁, R₂, R₃ et R₄ sont tels que définis ci-dessus,
- R₅ représente un atome d'hydrogène ou un groupe (C₁-C₄)alkyle ou -CO-((C₁-C₄)alkyle), et
- R₆ représente un atome d'hydrogène ou un groupe (C₁-C₄)alkyle,
en présence d'un thiol, à une température supérieure ou égale à 60°C, pour donner un composé de formule (I), et
(ii) séparation du composé de formule (I) obtenu à l'étape (i) précédente du milieu réactionnel.

2. Procédé selon la revendication 1, **caractérisé en ce que** le thiol répond à la formule R-SH, avec R représentant une chaîne alkyle, linéaire ou ramifiée, et de préférence linéaire, comportant de 1 à 8, notamment 2 à 6, atomes de carbone, substituée par un ou plusieurs groupes choisis parmi OH, SH, NH₂ et COOH.

3. Procédé selon la revendication 2, **caractérisé en ce que** le thiol est choisi parmi la cystéine, le dithiothréitol, le 2-mercaptoéthanol, l'acide 2-mercaptopropionique, l'acide 3-mercaptopropionique, l'acide mercaptoacétique, l'acide mercaptohexanoïque et l'acide thioglycolique, et de préférence est l'acide 3-mercaptopropionique.

4. Procédé selon la revendication 1, **caractérisé en ce que** l'étape (i) est réalisée à une température comprise entre 60 et 120°C, notamment entre 80 et 100°C.

5. Procédé selon l'une quelconque des revendications 1 à 4, **caractérisé en ce que** le composé de formule (I) répond à la formule (Ia) suivante : 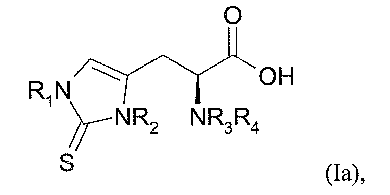 ou à un sel physiologiquement acceptable de celui-ci, à un tautomère, à un stéréoisomère ou à un mélange de stéréoisomères en toutes proportions, en particulier à un mélange d'énantiomères, et notamment à un mélange racémique de celui-ci,
pour laquelle R₁, R₂, R₃ et R₄ sont tels que définis à la revendication 1.

6. Procédé selon l'une quelconque des revendications 1 à 5, **caractérisé en ce que** le composé de formule (I) représente la 2-thiohistidine, l'α-méthyl-2-thiohistidine ou l'α,α-diméthyl-2-thiohistidine, et notamment la L-2-thiohistidine.

7. Procédé selon l'une quelconque des revendications 1 à 6, **caractérisé en ce que** le composé de formule (II) est préparé à partir d'un sel d'addition d'acide, à l'exclusion du sel de l'acide iodhydrique, du composé de formule (III) suivante : 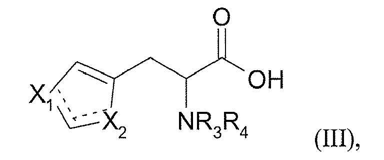 ou d'un tautomère, d'un stéréoisomère ou d'un mélange de stéréoisomères en toutes proportions, en particulier d'un mélange d'énantiomères, et notamment d'un mélange racémique de celui-ci,
pour laquelle 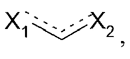 R₃ et R₄ sont tels que définis à la revendication 1,
par réaction successivement avec du dibrome,
puis avec un dérivé de cystéine de formule (IV) suivante : 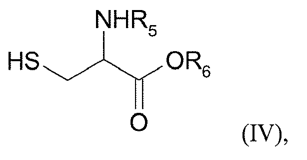 ou un stéréoisomère ou un mélange de stéréoisomères en toutes proportions, en particulier un mélange d'énantiomères, et notamment un mélange racémique de celui-ci,
dans laquelle R₅ et R₆ sont tels que définis à la revendication 1.

8. Procédé selon la revendication 7, **caractérisé en ce que** le composé de formule (I) répond à la formule (Ia) telle que définie à la revendication 5 et que le procédé comprend les étapes successives suivantes :
(a2) réaction d'un sel d'addition d'acide, à l'exclusion du sel de l'acide iodhydrique, d'un composé de formule (IIIa) suivante : 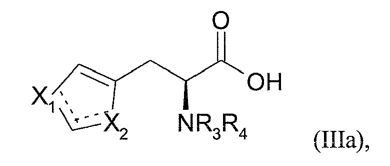 ou d'un stéréoisomère ou d'un mélange de stéréoisomères en toutes proportions, en particulier d'un mélange d'énantiomères, et notamment d'un mélange racémique de celui-ci,
pour laquelle 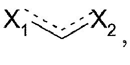 R₃ et R₄ sont tels que définis à la revendication 1, avec du dibrome,
puis avec un dérivé de cystéine de formule (IV) tel que défini à la revendication 7 ou un stéréoisomère ou un mélange de stéréoisomères en toutes proportions, en particulier un mélange d'énantiomères, et notamment un mélange racémique de celui-ci, et en particulier avec de la cystéine et notamment de la L-cystéine,
pour donner un composé de formule (IIa) suivante : 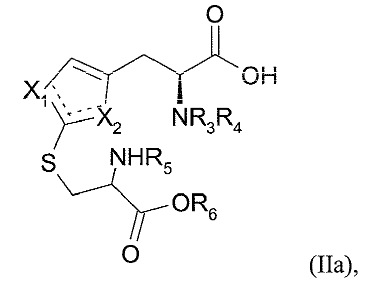 ou un sel physiologiquement acceptable, un tautomère un stéréoisomère ou un mélange de stéréoisomères en toutes proportions, en particulier un mélange d'énantiomères, et notamment un mélange racémique de celui-ci,
pour laquelle 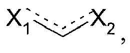 R₃, R₄, R₅ et R₆ sont tels que définis à la revendication 1,
(b2) réaction de clivage du composé de formule (IIa) obtenu à l'étape (a2) précédente en présence d'un thiol, à une température supérieure ou égale à 60°C, pour donner un composé de formule (Ia), et
(c2) séparation du composé de formule (Ia) obtenu à l'étape (b2) précédente du milieu réactionnel.

9. Procédé selon l'une quelconque des revendications 7 et 8, **caractérisé en ce que** le dérivé de cystéine de formule (IV) est utilisé en excès, à raison de 2 à 7, avantageusement 3 à 5 équivalents molaires de dérivé de cystéine par rapport au composé de formule (III).

10. Procédé selon l'une quelconque des revendications 7 à 9, **caractérisé en ce que** le dibrome est utilisé à raison de 1 à 1,5 équivalents molaires par rapport au composé de formule (III).

11. Procédé selon l'une quelconque des revendications 7 à 10, **caractérisé en ce que** la préparation du composé (I) à partir du composé (III) est réalisé dans un seul réacteur, sans isolation du composé (II) intermédiaire.

## Patentansprüche

1. Verfahren zur Synthese von einem Derivats von 2-Thiohistidin der nachfolgenden Formel (I): 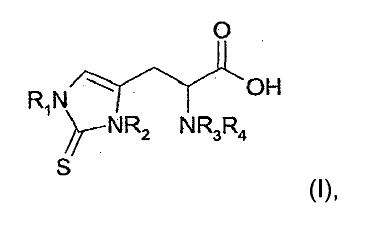 oder von einem physiologisch verträglichen Salz davon, einem Tautomer, einem Stereoisomer oder einem Gemisch aus Stereoisomeren in beliebigen Verhältnissen,
wobei
- R₁ und R₂ unabhängig voneinander ein Wasserstoffatom oder eine (C₁-C₄)-Alkylgruppe darstellen, wobei zumindest eine der Gruppierungen R₁ und R₂ ein Wasserstoffatom darstellt, und
- R₃ und R₄ unabhängig voneinander ein Wasserstoffatom oder eine (C₁-C₄)-Alkylgruppe darstellen,
umfassend die nachstehenden, aufeinanderfolgenden Schritte:
(i) Spaltungsreaktion einer Verbindung der nachfolgenden Formel (II): 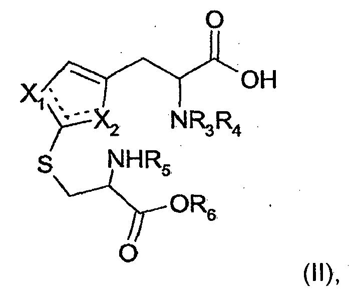 oder von einem physiologisch verträglichen Salz, einem Tautomer, einem Stereoisomer oder einem Gemisch aus Stereoisomeren in beliebigen Verhältnissen,
wobei
- 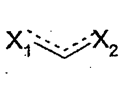 entweder 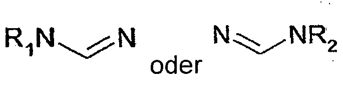 darstellt,
- R₁,R₂, R₃ und R₄ wie oben definiert sind,
- R₅ ein Wasserstoffatom oder eine (C₁-C₄)-Alkylgruppe oder -CO-((C₁-C₄)Alkylgruppe darstellt, und
- R₆ ein Wasserstoffatom oder eine (C₁-C₄)-Alkylgruppe darstellt,
unter Vorhandensein von Thiol bei einer Temperatur höher oder gleich 60 °C, um eine Verbindung der Formel (I) zu ergeben, und
(ii) Abtrennung der im vorangehenden Schritt (i) erhaltenen Verbindung der Formel (I) vom Reaktionsmedium.

2. Verfahren nach Anspruch 1, **dadurch gekennzeichnet, dass** das Thiol der Formel R-SH entspricht, wobei R eine lineare oder verzweigte, vorzugsweise eine lineare Alkylkette darstellt mit 1 bis 8, insbesondere 2 bis 6 Kohlenstoffatomen, substituiert mit einer oder mehreren Gruppen ausgewählt aus OH, SH, NH₂ und COOH.

3. Verfahren nach Anspruch 2, **dadurch gekennzeichnet, dass** das Thiol ausgewählt ist aus Cystein, Dithiothreitol, 2-Mercaptoethanol, 2-Mercaptopropionsäure, 3-Mercaptopropionsäure, Mercaptoessigsäure, Mercaptohexansäure und Thioglykolsäure, und vorzugsweise 3-Mercaptopropionsäure ist.

4. Verfahren nach Anspruch 1, **dadurch gekennzeichnet, dass** der Schritt (i) bei einer Temperatur zwischen 60 und 120 °C, insbesondere zwischen 80 und 100 °C, erfolgt.

5. Verfahren nach einem der Ansprüche 1 bis 4, **dadurch gekennzeichnet, dass** die Verbindung der Formel (I) der nachfolgenden Formel (la) entspricht: 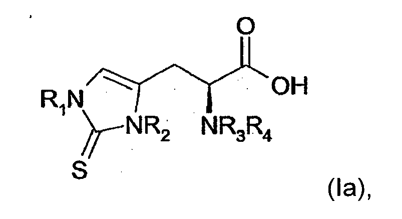 oder einem physiologisch verträglichen Salz davon, einem Tautomer, einem Stereoisomer oder einem Gemisch aus Stereoisomeren in beliebigen Verhältnissen, insbesondere einem Gemisch aus Enantiomeren und ganz besonders einem racemischen Gemisch davon entspricht,
wobei R₁,R₂, R₃ und R₄ wie in Anspruch 1 definiert sind.

6. Verfahren nach einem der Ansprüche 1 bis 5, **dadurch gekennzeichnet, dass** die Verbindung der Formel (I) 2-Thiohistidin, α-Methyl-2-Thiohistidin oder α,α-Dimethyl-2-Thiohistidin und insbesondere L-2-Thiohistidin darstellt.

7. Verfahren nach einem der Ansprüche 1 bis 6, **dadurch gekennzeichnet, dass** die Verbindung der Formel (II) aus einem Säureadditionssalz, davon ausgeschlossen das Salz von Jodwasserstoffsäure, der Verbindung der nachfolgenden Formel (III) hergestellt wird: 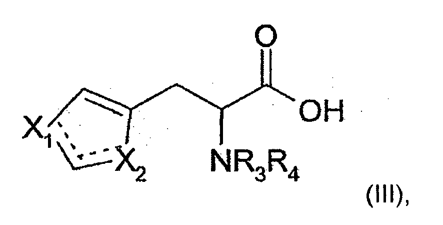 oder aus einem Tautomer, einem Stereoisomer oder einem Gemisch aus Stereoisomeren in beliebigen Verhältnissen, insbesondere einem Gemisch aus Enantiomeren und ganz besonders aus einem racemischen Gemisch desselben,
wobei 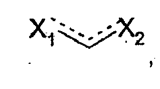 R₃ und R₄ wie in Anspruch 1 definiert sind,
durch aufeinanderfolgende Reaktion mit Dibrom,
dann mit einem Cystein-Derivat der nachfolgenden Formel (IV): 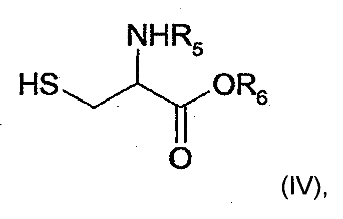 oder einem Stereoisomer oder einem Gemisch aus Stereoisomeren in beliebigen Verhältnissen, insbesondere einem Gemisch aus Enantiomeren und ganz besonders aus einem racemischen Gemisch desselben,
wobei R₅ und R₆ wie in Anspruch 1 definiert sind.

8. Verfahren nach Anspruch 7, **dadurch gekennzeichnet, dass** die Verbindung der Formel (I) der Formel (la) wie in Anspruch 5 definiert entspricht und dass das Verfahren die nachstehenden aufeinanderfolgenden Schritte umfasst:
(a2) Reaktion eines Säureadditionssalzes, davon ausgeschlossen das Salz von Jodwasserstoffsäure, von einer Verbindung der nachfolgenden Formel (IIIa): 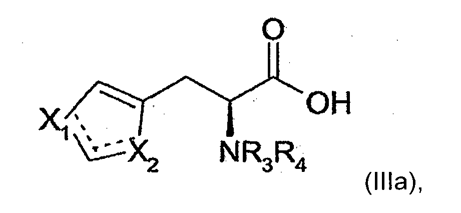 oder einem Stereoisomer oder einem Gemisch aus Stereoisomeren in beliebigen Verhältnissen, insbesondere einem Gemisch aus Enantiomeren und ganz besonders aus einem racemischen Gemisch desselben,
wobei 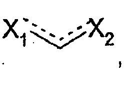 R₃ und R₄ wie in Anspruch 1 definiert sind,
mit Dibrom,
dann mit einem Cystein-Derivat der Formel (IV) wie in Anspruch 7 definiert oder einem Stereoisomer oder einem Gemisch aus Stereoisomeren in beliebigen Verhältnissen, insbesondere einem Gemisch aus Enantiomeren und ganz besonders aus einem racemischen Gemisch desselben und insbesondere mit Cystein und ganz insbesondere mit L-Cystein,
um eine Verbindung der nachfolgenden Formel (IIa) zu ergeben: 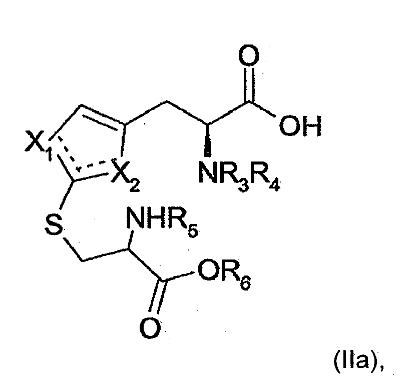 oder ein physiologisch verträgliches Salz, ein Tautomer, ein Stereoisomer oder ein Gemisch aus Stereoisomeren in beliebigen Verhältnissen, insbesondere ein Gemisch aus Enantiomeren und ganz besonders ein racemisches Gemisch desselben,
wobei 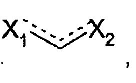 R₃, R₄, R₅ und R₆ wie in Anspruch 1 definiert sind,
(b2) Spaltungsreaktion der im vorangehenden Schritt (a2) erhaltenen Verbindung der Formel (IIa) unter Vorhandensein von einem Thiol bei einer Temperatur höher oder gleich 60 °C, um eine Verbindung der Formel (la) zu ergeben, und
(c2) Abtrennung der im vorangehenden Schritt (b2) erhaltenen Verbindung der Formel (la) vom Reaktionsmedium.

9. Verfahren nach einem der Ansprüche 7 und 8, **dadurch gekennzeichnet, dass** in Bezug auf die Verbindung der Formel (III) das Cystein-Derivat der Formel (IV) im Überschuss mit 2 bis 7, vorteilhaft mit 3 bis 5 Moläquivalenten von Cystein-Derivat verwendet wird.

10. Verfahren nach einem der Ansprüche 7 bis 9, **dadurch gekennzeichnet, dass** in Bezug auf die Verbindung der Formel (III) das Dibrom mit 1 bis 1,5 Moläquivalenten verwendet wird.

11. Verfahren nach einem der Ansprüche 7 bis 10, **dadurch gekennzeichnet, dass** die Herstellung der Verbindung (I) ausgehend von der Verbindung (III) in einem einzigen Reaktor ohne Isolierung der Zwischenverbindung (II) erfolgt.

## Claims

1. A method for the synthesis of a 2-thiohistidine derivative of the following formula (I): 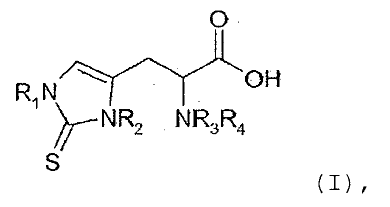 or a physiologically acceptable salt thereof, a tautomer, a stereoisomer or a mixture of stereoisomers in any proportions,
wherein:
- R₁ and R₂ represent, independently of each other, a hydrogen atom or a (C₁-C₄)alkyl group, at least one of the groups R₁ and R₂ representing a hydrogen atom, and
- R₃ and R₄ represent, independently of each other, a hydrogen atom or a (C₁-C₄)alkyl group,
comprising the following successive steps:
(i) cleavage reaction of a compound of following formula (II): 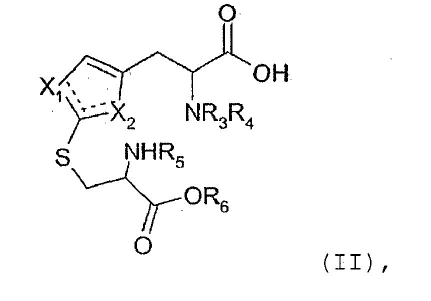 or a physiologically acceptable salt, a tautomer, a stereoisomer or a mixture of stereoisomers in any proportions, wherein:
- 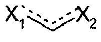 represents 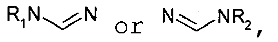
- R₁, R₂, R₃ and R₄ are such as defined above,
- R₅ represents a hydrogen atom or a (C₁-C₄) alkyl or -CO-((C₁-C₄)alkyl) group, and
- R₆ represents a hydrogen atom or a (C₁-C₄) alkyl group,
in the presence of a thiol at a temperature higher than or equal to 60°C, to yield a compound of formula (I), and
(ii) separation of the compound of formula (I) obtained in the preceding step (i) from the reaction medium.

2. The method according to claim 1, **characterized in that** the thiol is of formula R-SH, with R representing a linear or branched, preferably linear, alkyl chain comprising from 1 to 8, notably 2 to 6, carbon atoms, substituted by one or more groups selected from OH, SH, NH₂ and COOH.

3. The method according to claim 2, **characterized in that** the thiol is selected from cysteine, dithiothreitol, 2-mercaptoethanol, 2-mercaptopropionic acid, 3-mercaptopropionic acid, mercaptoacetic acid, mercaptohexanoic acid and thioglycolic acid, and preferably is 3-mercaptopropionic acid.

4. The method according to claim 1, **characterized in that** the step (i) is carried out at a temperature between 60°C and 120°C, notably between 80°C and 100°C.

5. The method according to any one of claims 1 to 4, **characterized in that** the compound of formula (I) has the following formula (Ia): 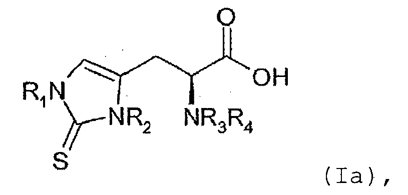 or a physiologically acceptable salt thereof, a tautomer, a stereoisomer or a mixture of stereoisomers in any proportions, in particular a mixture of enantiomers, and notably a racemic mixture thereof,
wherein R₁, R₂, R₃ and R₄ are such as defined in claim 1.

6. The method according to any one of claims 1 to 5, **characterized in that** the compound of formula (I) represents 2-thiohistidine, α-methyl-2-thiohistidine or α,α-dimethyl-2-thiohistidine, and notably L-2-thiohistidine.

7. The method according to any one of claims 1 to 6, **characterized in that** the compound of formula (II) is prepared from an acid addition salt, to the exclusion of a hydriodic acid salt, of the compound of the following formula (III): 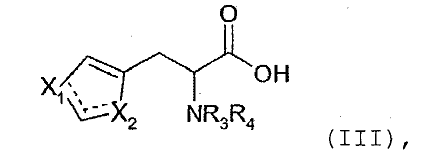 or a tautomer or a stereoisomer or a mixture of stereoisomers in any proportions, in particular a mixture of enantiomers, and notably a racemic mixture thereof,
wherein 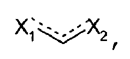 R₃ and R₄ are such as defined in claim 1,
by successive reaction with bromine,
and then with a cysteine derivative of the following formula (IV): 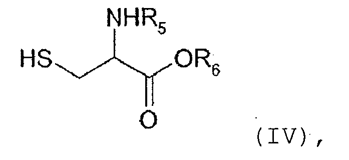 or a stereoisomer or a mixture of stereoisomers in any proportions, in particular a mixture of enantiomers, and notably a racemic mixture thereof,
wherein R₅ and R₆ are such as defined in claim 1.

8. The method according to claim 7, **characterized in that** the compound of formula (I) has the formula (Ia) as defined in claim 5 and **in that** the method comprises the following successive steps:
(a2) reaction of an acid addition salt, to the exclusion of a hydriodic acid salt, of a compound of the following formula (IIIa): 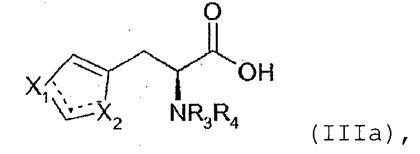 or a stereoisomer or a mixture of stereoisomers in any proportions, in particular a mixture of enantiomers, and notably a racemic mixture thereof,
wherein 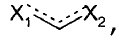 R₃ and R₄ are such as defined in claim 1,
with bromine,
and then with a cysteine derivative of the formula (IV) such as defined in claim 7 or a stereoisomer or a mixture of stereoisomers in any proportions, in particular a mixture of enantiomers, and notably a racemic mixture thereof, and in particular with cysteine and notably L-cysteine,
to yield a compound of the following formula (IIa) : 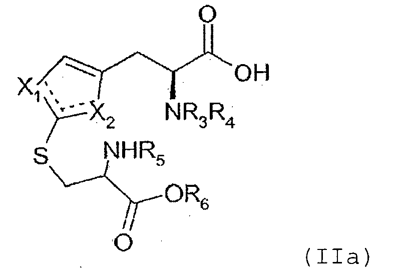 or a physiologically acceptable salt, a tautomer, a stereoisomer or a mixture of stereoisomers in any proportions, in particular a mixture of enantiomers, and notably a racemic mixture thereof,
wherein 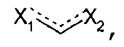 R₃, R₄, R₅ and R₆ are such as defined in claim 1,
(b2) cleavage reaction of the compound of formula (IIa) obtained in the preceding step (a2) in the presence of a thiol, at a temperature higher than or equal to 60°C, to yield a compound of formula (Ia), and
(c2) separation of the compound of formula (Ia) obtained in the preceding step (b2) from the reaction medium.

9. The method according to any one of claims 7 and 8, **characterized in that** the cysteine derivative of formula (IV) is used in excess, in a ratio of 2 to 7, advantageously 3 to 5, molar equivalents of the cysteine derivative in relation to the compound of formula (III).

10. The method according to any one of claims 7 to 9, **characterized in that** bromine water is used in a ratio of 1 to 1.5 molar equivalents in relation to the compound of the formula (III).

11. The method according to any one of claims 7 to 10, **characterized in that** the preparation of compound (I) from compound (III) is carried out in a single reactor, without isolation of the intermediate compound (II).
